# EUROPEAN PATENT SPECIFICATION

(11) **EP 3 866 873 B1**
(45) Date of publication and mention of the grant of the patent: **02.12.2026**
(21) Application number: 19870051.0
(22) Date of filing: 15.10.2019
(51) Int. Cl.: A61M 1/02

(54) **A METHOD FOR OBTAINING PLATELET RICH PLASMA**
VERFAHREN ZUR GEWINNUNG VON PLÄTTCHENREICHEM PLASMA
PROCÉDÉ D'OBTENTION DE PLASMA RICHE EN PLAQUETTES

(30) Priority: 18.10.2018 TR 201815514
(43) Date of publication of application: 25.08.2021
(73) Proprietor: T-BIYOTEKNOLOJI LABORATUVAR ESTETIK MEDIKAL KOZMETIK SAN TIC LTD STI, 16240 Nilüfer / Bursa (TR)
(72) Inventor: DOGRUOK, Timur Veysel, Nilüfer/ Bursa (TR); ÖZOVALI, Salih, Nilüfer/ Bursa (TR)
(86) International application number: PCT/TR2019/050870
(87) International publication number: WO 2020/081036

(56) References cited:
- DE-A1- 102011 050 836
- DE-A1- 102013 003 326
- US-A1- 2010 288 706
- US-A1- 2018 110 917
- US-B2- 8 168 230

## Description

### TECHNICAL FIELD

The invention is a method for obtaining platelet-rich plasma.

The invention is particularly related to a centrifuge method carried out during said method of obtaining platelet-rich plasma in order to delay the coagulation period without using an anticoagulant.

### PRIOR ART

The platelet-rich plasma known as PRP, is used in several medical fields such as plastic surgery, dentistry, orthopedics and dermatology for tissue healing or to increase the rate of tissue healing. Platelet-rich plasma is usually applied directly or with the aid of a syringe to the damaged surface where healing is desired.

Platelet-rich plasma, is extracted from blood, however it comprises a growth factor which enables better tissue healing in comparison to blood.

In order to extract the platelet rich plasma from the blood, the red blood cells contained in the blood and the platelet plasma are separated from each other by centrifugation of the blood at a certain angle received in a tube. Following centrifuge, blood is separated as layers inside the tube and platelet-rich plasma can be drawn on its own from the related tube.

As it is known; blood has a structure that coagulates very rapidly outside the body. Coagulation starts approximately within 3-4 minutes following contact with air. One of the factors that causes coagulation is thrombocytes. Thrombocytes interact with free calcium ions and they support the formation of clot.

The centrifugal force created during the centrifuging process of blood, causes the platelets to hit the side walls of the centrifuge tubes, thereby damaging platelet membranes. During this event, protruding pseudopodias emerge and this accelerates the coagulation process.

In order to prevent rapid coagulation and gelation of the obtained platelet-rich plasma, sodium citrate or a similar anticoagulant agent is added to blood following or during the taking of blood from a donor. Anticoagulant prevents the free calcium ions and platelets get interacted each other which this chemical reaction prevents the coagulation.

Usage of an anticoagulant agent also brings about various problems.

Although it is an obligation to apply foreign material such as anticoagulants to a body according to the prior art, it is actually an unwanted situation. Besides this, the presence of an anticoagulant agent, causes platelet-rich plasma treatment to be classified as blood transfusion by some local authorities and leads to impose the regulations of said classification to this method.

Another problem is that the usage of anticoagulants slows down said process. Under normal conditions, blood is taken from the donor by using syringes made of polypropylene (PP) and other similar materials. PP, absorbs the anticoagulant, therefore said syringes cannot be sold including anticoagulants ready for use. A certain amount of anticoagulants are added into the syringe either before or after phlebotomy

Following the preliminary patent search, the below mentioned documents have been encountered.

In the USPTO document with the publication number US2014360944 (A1), a method and a system used to obtain plasma that is platelet-rich plasma is disclosed. In the method one of the basic steps has been explained as the addition of an anticoagulant agent before the centrifuge process.

In the USPTO document with the publication number US8168230 (B2), a method to obtain platelet-rich plasma is disclosed. The example given in said document is as follows: 45 ml venous blood is taken from a patient using a syringe comprising 5 ml and 0.38% sodium citrate. The whole blood is centrifuged in a swinging bucket type centrifuge device for 20 minutes at 180 G centrifugal force in order to separate the red blood cells from the platelet plasma. The blood residues of red blood cells are drawn out by means of a canula placed on the stopper of the centrifuge tube and following this platelet plasma is re-centrifuged for 15 more minutes at 580 G centrifugal force in order to separate the platelet sediment from the platelet-poor plasma.

As a result all of the problems mentioned above have made it necessary to provide a novelty in the related field.

### AIM OF THE INVENTION

The present invention aims to eliminate the problems mentioned above and to provide a novelty in the related technical field.

The main aim of the invention is particularly related to providing a centrifuge method carried out during said method for delaying the coagulation process of the obtained platelet-rich plasma without using an anticoagulant.

Another aim of the invention is to eliminate the usage of an anticoagulant in the method of obtaining the related platelet-rich plasma.

Another aim of the invention is to obtain platelet-rich plasma that does not contain an anticoagulant.

Another aim of the invention is to allow the usage of kits made of different materials such as PP in order to obtain platelet-rich plasma from a donor.

### BRIEF DESCRIPTION OF THE INVENTION

The present invention that is mentioned above and shall be construed from the detailed description below, is a method to obtain platelet-rich plasma that has not coagulated and which does not comprise an anticoagulant. Accordingly, centrifuging blood in a container (22), that does not contain anticoagulants, wherein the container (22) is held within a carrier of a swinging bucket type centrifugation device (1), and said centrifuging is carried out parallel to the ground for a duration of 2 to 6 minutes and such that it generates between 1400 and 1500 G relative centrifugal force in order to prevent damaging membranes of the blood platelets due to said platelets inside the blood hitting the side surface (22.a) of the container (22), wherein the carrier (20) rotates around a spindle (111) axis, ensuring the container (22) remains parallel to the ground throughout the centrifugation process.

According to a preferred embodiment of the invention, said centrifuge process is carried out for 4 minutes.

According to a another preferred embodiment of the invention, said centrifuge process is provided to generate 1500 G / relative centrifugal force.

### DESCRIPTION OF THE FIGURES:

Figure 1 is the microscopic view of a passive platelet cell.
Figure 1A is the microscopic view of an activated platelet cell.
Figure 2, is the illustrative schematic front view of an embodiment of a centrifuge device to be used in the method subject to the invention.
Figure 2A, is the illustrative schematic top view of an embodiment of a centrifuge device to be used in the method subject to the invention.

### DESCRIPTION OF THE REFERENCE NUMBERS IN THE FIGURES

**1.** Swinging bucket type centrifuge device
**10.** Body
**11.** Arms
**111.** Spindle
**12.** Shaft
**20.** Carrier
**21.** Slot
**22.** Housing
**22.a.** Side surface
**22.b.** Base

### DETAILED DESCRIPTION OF THE INVENTION

In this detailed description, the method for obtaining platelet-rich plasma according to the invention has been described with examples that shall not be construed to limit the invention but have been provided in order to better explain the subject matter of the invention.

The subject of the invention is related to a centrifuge method carried out during said method of obtaining platelet-rich plasma in order to prevent the coagulation of the obtained platelet-rich plasma.

Blood taken from a donor in order to obtain platelet-rich plasma, is added into a container (22). Said container (22) can be a tube or it can be a kit where blood is directly drawn from the donor.

The invention is related to a method for obtaining platelet-rich plasma that has not coagulated and which does not comprise an anticoagulant, characterized in that;
centrifuging blood in a container (22), that does not contain anticoagulants and said centrifuging is carried out parallel to the ground for at least 2 minutes and such that it generates at least 350 G / relative centrifugal force in order to prevent damaging membranes of the blood platelets due to said platelets inside the blood hitting the side surface (22.a) of the container (22).

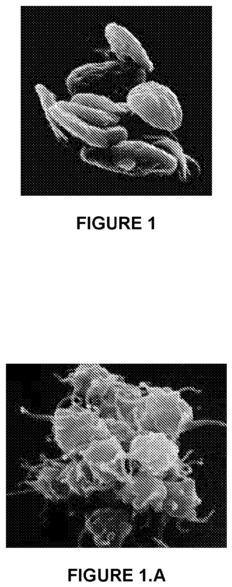

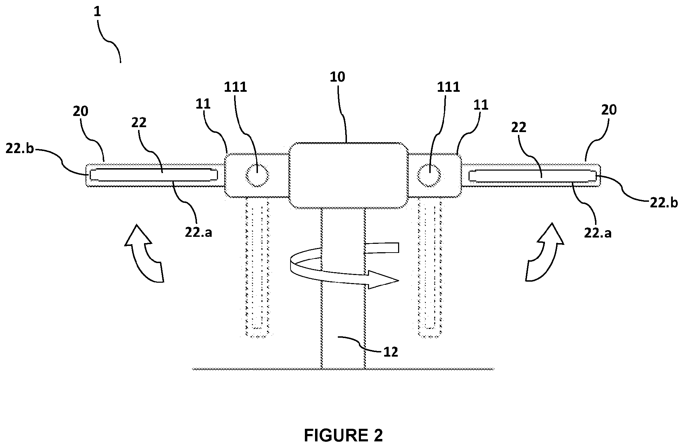

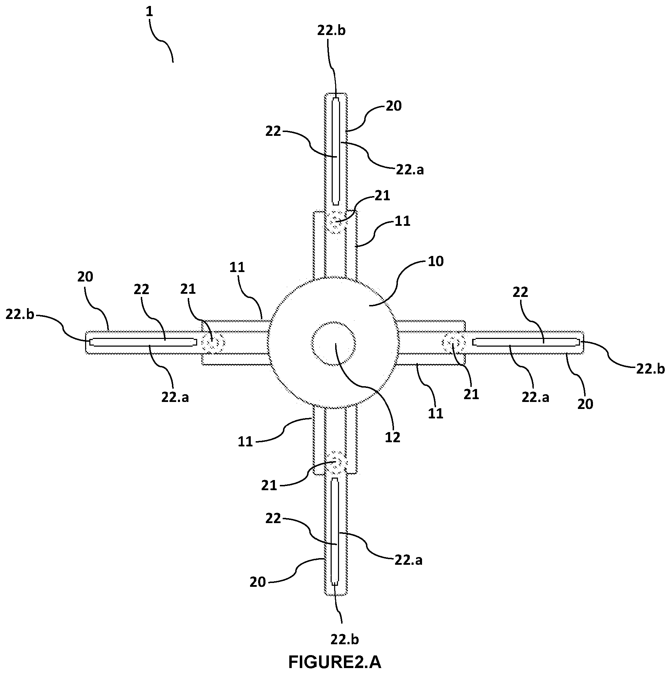

During the inclined centrifugation process, platelets are separated from the red blood cells in a similar way and they accumulate at the base (22.b) section of the container (22) where centrifuge is provided. However, the fixed direction of centrifugal force and the centrifuge process, causes said platelets to hit the side surface (22.a) of the container (22) before reaching the base (22.b). The hitting of the platelets to the side wall damages the platelet membrane and this accelerates the transition process of the platelets from the passive state, as shown in Figure 1, to the active state as shown in Figure 2.

In the method subject to the invention by providing the centrifuge, parallel to the ground, the separation direction of the platelet-rich plasma and the centrifugal force are enabled to be in the same direction. The platelet-rich plasma comprises an amount of red blood cells. Red blood cells that are larger than other components settle on the base (22.b) of the container (22) before the platelets and therefore they create a bed which reduces the impact effect for platelets. As a result platelet membranes are damaged, or in other words they maintain their structure shown in Figure 1 and the acceleration in coagulation seen in the prior art, is not observed.

In the method subject to the invention centrifuging is carried out for at least 2 minutes, such that it generates at least 350 G / relative centrifugal force. The amount of the red blood cells, white blood cells and platelet ratios inside platelet-rich plasma obtained by increasing the centrifugal force may vary. Said ratios may vary among blood donors. The amount of the platelet-rich plasma, and the red blood cells, white blood cells and platelet ratios obtained from the blood samples of different donors have been provided in the Table below.

**TABLE 1**

| **ID** | **RCF** | **Plasma ml** | **Whole Blood PLT Number** | **Enriched PLT Number** | **Enrichment Coefficient** | **RBC** | **WBC** |
|---|---|---|---|---|---|---|---|
| 1 | 1500 | 4,5 | 248 | 326 | 1,31 | 0,00 | 0,48 |
| 2 | 1500 | 5,5 | 168 | 470 | 2,80 | 0,00 | 2,06 |
| 3 | 1400 | 5 | 276 | 534 | 1,93 | 0,02 | 1,08 |
| 4 | 1600 | 6 | 173 | 203 | 1,17 | 0,01 | 0,51 |
| 5 | 1000 | 4 | 223 | 488 | 2,19 | 0,04 | 8,09 |
| 5 | 800 | 6 | 231 | 357 | 1,55 | 0,02 | 3,99 |
| 6 | 400 | 2,5 | 471 | 1042 | 2,21 | 0,09 | 8,54 |
| 7 | 1400 | 5,5 | 298 | 397 | 1,33 | 0,01 | 1,49 |
| 8 | 1300 | 6 | 262 | 298 | 1,14 | 0,01 | 0,49 |
| 9 | 1400 | 5 | 276 | 526 | 1,91 | 0,02 | 3,85 |
| 10 | 1000 | 3,5 | 223 | 500 | 2,24 | 0,05 | 8,94 |
| 11 | 1000 | 5 | 390 | 600 | 1,54 | 0,03 | 5,42 |

The results obtained in the assays carried out using the above described method on plasmas obtained from different donors are shown in Table 1. The amount of plasma and the platelet amount in blood may vary from donor to donor. The term "RCF" expresses the G Force, applied to the plasma during centrifuge, "RBC" expresses the red blood cell rate in platelet-rich plasma, "WBC" expresses the white blood cell rate in platelet-rich plasma. Although enrichment rate of platelets are high even at low RCF values, it can be seen that as RCF value increases the undesired WBC rate decreases. Accordingly the coagulation time and WBC rate are provided between 1400-1500 G.

The platelet-rich plasma obtained by using the related method, can remain in usable form up to approximately 30 minutes.

Figure 2, is the illustrative schematic front view of an embodiment of a centrifuge device to be used in the method subject to the invention and Figure 2A, is the illustrative schematic top view of an embodiment of a centrifuge device to be used in the method subject to the invention.

A swinging bucket type centrifuge device (1) is used in said method in order to centrifuge the blood. The swinging bucket type centrifuge device (1) basically comprises a body (10) rotated via a shaft (12) by means of a drive system, arms (11) attached to the body (10), and a carrier (20) coupled to the arms (11) via a spindle (111), where said carrier is configured to rotate around the axis of said spindle (111). Said carrier (20) comprises a slot (21) for placing the container (22) therein.

The carriers (20) has been positioned vertical to the ground when the device is off. The related position has been shown with dashed lines in Figure 2. In said system, by means of the drive provided on the shaft (12), the centrifuge process is started and the swinging bucket type centrifuge device (1) starts to rotate around the shaft (12) axis. The centrifugal force created by means of the rotational motion, enables the carriers (20) to rotate around the spindle (111) axis and the movement at the arrow direction in Figure 2 is created and this enables the carrier (20) to be positioned parallel to the ground which in turn provides the required position for the method subject to the invention.

The protection scope of the invention has been defined in the attached claims and this scope cannot be limited to the examples disclosed in the description document. It is obvious that the person skilled in the art can convey similar embodiments in the light of the information given above without deviating from the main theme of the invention.

## Claims

1. A method of obtaining platelet-rich plasma that has not coagulated and which does not comprise an anticoagulant, **comprising the steps of:**
• centrifuging blood in a container (22) that does not contain anticoagulants, wherein the container (22) is held within a carrier (20) of a swinging bucket type centrifuge device (1);
• said centrifuging being carried out parallel to the ground for a duration of 2 to 6 minutes, such that it generates a relative centrifugal force between 1400-1500 G to prevent damaging the membranes of blood platelets due to the platelets inside the blood hitting the side surface (22.a) of the container (22), wherein the carrier (20) rotates around a spindle (111) axis, ensuring the container (22) remains parallel to the ground throughout the centrifugation process.

## Patentansprüche

1. Verfahren zur Gewinnung von nicht koaguliertem plättchenreichem Plasma, das kein Antikoagulans umfasst, **umfassend die Schritte:**
• Zentrifugieren von Blut in einem Behälter (22), der keine Antikoagulanzien enthält, wobei der Behälter (22) innerhalb eines Trägers (20) einer Zentrifugenvorrichtung (1) vom Typ einer Schwenkbecherzentrifuge gehalten wird;
• wobei das Zentrifugieren parallel zum Boden für eine Dauer von 2 bis 6 Minuten durchgeführt wird, derart, dass es eine relative Zentrifugalkraft zwischen 1400-1500 G erzeugt, um eine Beschädigung der Membranen von Blutplättchen dadurch zu verhindern, dass die in dem Blut enthaltenen Blutplättchen auf die Seitenfläche (22.a) des Behälters (22) auftreffen, wobei sich der Träger (20) um die Achse einer Spindel (111) dreht, wodurch sichergestellt wird, dass der Behälter (22) während des gesamten Zentrifugationsvorgangs parallel zum Boden bleibt.

## Revendications

1. Méthode d'obtention d'un plasma riche en plaquettes qui n'a pas coagulé et qui ne contient pas d'anticoagulant, **comprenant les étapes suivantes** :
• centrifuger du sang dans un récipient (22) ne contenant pas d'anticoagulants, dans lequel le récipient (22) est maintenu à l'intérieur d'un support (20) d'un dispositif centrifuge (1) de type à godets oscillants;
• ladite centrifugation étant effectuée parallèlement au sol pendant une durée de 2 à 6 minutes, de manière à générer une force centrifuge relative comprise entre 1400-1500 G afin d'éviter d'endommager les membranes des plaquettes sanguines dû au heurt des plaquettes à l'intérieur du sang contre la surface latérale (22.a) du récipient (22), dans lequel le support (20) tourne autour d'un axe de broche (111), garantissant que le récipient (22) reste parallèle au sol tout au long du processus de centrifugation.
